# EUROPÄISCHE PATENTANMELDUNG

(11) **EP 0 976 759 A2**
(43) Veröffentlichungstag der Anmeldung: **02.02.2000**
(21) Anmeldenummer: 99113359.6
(22) Anmeldetag: 09.07.1999
(51) Int. Cl.: C07K 1/34, C07K 14/745, C07K 14/765, C07K 16/06

(54) **Herstellung von Proteinpräparationen mit verringertem Aggregatgehalt**

(30) Priorität: 10.07.1998 DE 19831061
(71) Anmelder: ZLB Zentrallaboratorium Blutspendedienst SRK, CH-3000 Bern 22 (CH)
(72) Erfinder: Förtsch, Verena, 4600 Olten (CH); Reichen, Kurt, 3703 Aeschi (CH); Lerch, Peter G., 3007 Bern (CH)
(74) Vertreter: Weiss, Wolfgang, Dipl.-Chem. Dr.

(57) **Zusammenfassung**

Die Erfindung betrifft ein Verfahren zur Herstellung von Proteinpräparationen mit verringertem Aggregatgehalt, insbesondere zur Herstellung von medizinischen Präparationen von Blutplasmaproteinen wie etwa Albumin.

## Beschreibung

Die Erfindung betrifft ein Verfahren zur Herstellung von Proteinpräparationen mit verringertem Aggregatgehalt, insbesondere zur Herstellung von medizinischen Präparationen von Blutplasmaproteinen wie etwa Albumin.

Humane Plasmaproteine werden seit einigen Jahrzehnten in großem Maßstab gereinigt und für die Therapie und Prophylaxe zugänglich gemacht. Für die Präparation von reinen Proteinen oder von Proteinfraktionen aus einem komplexen Plasmagemisch können verschiedene Methoden eingesetzt werden wie etwa Fraktionierung mittels selektiver Präzipitation oder Auftrennung der Proteingemische mittels chromatographischer Methoden wie Ionenaustausch-Chromatographie, Gelfiltration und Affinitätschromatographie. Um optimale Ergebnisse erhalten zu können, werden diese Methoden sehr oft auch kombiniert.

Für die Fraktionierung von Plasmaproteinen im großen Maßstab haben sich seit langer Zeit auch Fällungsmethoden mit Ethanol bewährt (Cohn et al., J. Am. Chem. Soc. 68 (1946), 459-475; Kistler und Nitschmann, Vox Sang. 7 (1962), 414-424). Mit dieser Methodik werden auch heute noch weltweit große Mengen von Plasmaproteinen, insbesondere Albumin, Immunglobuline und Gerinnungsfaktoren, aber auch weitere Proteine aus humanem Plasma vor allem für medizinische Zwecke isoliert. Die Ethanolfraktionierung von Plasmaproteinen hat jedoch einige Nachteile. So kann insbesondere bei hohen Alkoholkonzentrationen oder/und hohen Temperaturen eine teilweise Denaturierung von empfindlichen Proteinen erfolgen. Eine solche Denaturierung kann zum teilweisen oder vollständigen Verlust der physiologischen Funktion oder zu strukturellen Veränderungen führen, welche sich in der Aktivierung von Proenzymen oder der Bildung von neuen Antigendeterminanten oder Proteinaggregaten äußern können.

Um mögliche infektiöse Kontaminationen, z.B. Viren oder andere Pathogene, zu inaktivieren, können Blutplasmapräparationen einem abschließenden Pasteurisierungsschritt unterzogen werden. Albumin kann beispielsweise durch Erhitzen auf 60 bis 64°C für 10 h in Gegenwart von Stabilisatoren wie etwa N-Acetyltryptophanat oder Natriumcaprylat pasteurisiert werden (Gellis et al., J. Clin. Invest. 27 (1948), 239-244). Weitere Pasteurisierungsverfahren werden beispielsweise in den US-Patenten 2,897,123; 3,227,626; 4,379,085; 4,440,679; 4,623,717 und 4,803,073 offenbart. Pasteurisierte Präparationen von Plasmaproteinen haben sich bezüglich der Übertragung von Viren und Pathogenen als sehr sicher erwiesen. Ein weiterer Vorteil der Pasteurisierung besteht darin, daß der Proteinpräparation keinetoxischen Zusatzstoffe beigegeben werden müssen und somit in vielen Fällen eine Inaktivierung von Pathogenen im Endbehälter machbar ist. Ein Nachteil der Pasteurisierung besteht jedoch darin, daß oftmals ein deutlicher Anstieg der Aggregatbildung gefunden wird.

Diese Aggregatbildung, die zum Entstehen von visuellen oder subvisuellen Partikeln in teilweise hohen Mengen führt, ist jedoch in höchstem Maße unerwünscht, insbesondere in medizinischen und pharmazeutischen Anwendungen. So können große Partikel direkt die Funktion von Kapillargefäßen beeinträchtigen. Unerwünschte Nebenwirkungen von subvisuellen Partikeln oder Proteinaggregaten sind für die verschiedensten Proteinformulierungen bekannt und beschrieben worden. Deshalb bestehen die meisten Zulassungsbehörden auf Grenzwerten für den Aggregatgehalt, beispielsweise von Albumin- oder Immunglobulinlösungen. Aggregate in Immunglobulinlösungen können etwa eine unkontrollierte Aktivierung des Komplementsystems auslösen und zu schwerwiegenden Nebenwirkungen führen. Von Albuminaggregaten ist beschrieben, daß sie sehr schnell aus der Zirkulation verschwinden, wahrscheinlich das RES-System blockieren und den Organismus für Schockzustände sensibilisieren. Somit können Aggregate in Proteinlösungen im Extremfall zu lebensbedrohenden Situationen führen, welche unter allen Umständen vermieden werden müssen.

Wegen des sehr breiten Anwendungsgebiets von Plasmaproteinen besteht ein sehr hoher Bedarf an Verfahren, mit denen die Sicherheit, Verträglichkeit und die biologische Aktivität der Proteine verbessert werden kann. Zusätzlich sollten solche Verfahren wirtschaftlich und einfach anzuwenden sein.

Überraschenderweise wurde gemäß vorliegender Erfindung festgestellt, daß Proteinpäparationen mit verringertem Aggregatgehalt hergestellt werden können, wenn eine z.B. zur Beseitigung möglicher infektiöser Kontaminationen durchgeführte thermische Behandlung durch einen vorangehenden oder nachfolgenden Abtrennungsschritt verbessert wird, so daß im Endprodukt die Denaturierung oder/und Aggregatbildung signifikant reduziert oder verhindert ist. Durch den Abtrennungsschritt wird ein Protein oder Proteingemisch, welches anschließend thermisch behandelt werden soll, bezüglich seinem gewünschten aktiven und nativem Wirkstoff angereichert. Ein Gegenstand der vorliegenden Erfindung ist somit ein Verfahren zur Herstellung von Proteinpräparationen mit verringertem Aggregatgehalt umfassend eine thermische Behandlung, wobei das Verfahren dadurch gekennzeichnet ist, daß vor oder/und nach der thermischen Behandlung eine Abtrennung von in der Proteinpräparation vorhandenen Aggregaten, denaturierten Proteinen oder/und Kontaminationen erfolgt.

Das erfindungsgemäße Verfahren ist insbesondere zur Herstellung von Präparationen von Blutplasmaproteinen geeignet, jedoch nicht darauf beschränkt. Die Blutplasmaproteine werden vorzugsweise ausgewählt aus der Gruppe bestehend aus Plasminogen, Albumin, Faktor VIII, Faktor IX, Fibronektin, Immunoglobulinen, Kininogen, AntithrombinIII, α-1-Antitrypsin, Präkallikrein, Fibrinogen, Thrombin, (Apo-)-Lipoproteinen und Blutplasmaproteinfraktionen, die ein oder mehrere dieser Proteine enthalten. Besonders bevorzugt stellt man eine Präparation von Albumin, Immunglobulinen oder (Apo-)Lipoproteinen und am meisten bevorzugt eine Albuminpräparation her.

Eine durch das erfindungsgemäße Verfahren hergestellte Albuminpräparation mit verringertem Aggregatgehalt ist erheblich besser für medizinische Anwendungen geeignet als Präparate des Standes der Technik. Die erfindungsgemäßen Albuminpräparationen können als Plasmaexpander, aber auch für die Therapie bei Verbrennungen eingesetzt werden. Darüber hinaus sind auch weitere Anwendungen möglich, wie etwa das Ultraschall-Imaging oder der Zusatz als Stabilisator zu Proteinlösungen, insbesondere wenn es sich dabei um hochaktive Proteine oder Peptide in geringen Konzentrationen handelt, wie etwa Hormone, Chemokine, Zytokine oder Enzyme, die heute oft mit rekombinanter Technologie hergestellt werden.

Besonders ist das erfindungsgemäße Verfahren auch zur Herstellung von Präparationen chemisch modifizierter Proteine geeignet, wobei die Modifikation an funktionellen Gruppen der Proteine, insbesondere an funktionellen Seitenketten, durchgeführt wird. Chemisch modifizierte Proteine, z.B. Albumin, können beispielsweise als Träger von funktionellen Molekülen eingesetzt werden, die in vivo nicht in die gewünschten Kompartimente des Organismus gelangen oder in freier Form nicht die gewünschte Aktivität aufweisen. Vorzugsweise wird die chemische Modifizierung ausgewählt aus der Gruppe bestehend aus Polyethylenglykol-Modifizierung, Iodierung, Acylierung, z.B. Acetylierung, Oxidation, z.B. mit Peroxiden, Nitroxylierung und Quervernetzung, z.B. mit bifunktionellen Linkern.

Der für das erfindungsgemäße Verfahren wesentliche Schritt, d.h. eine mindestens teilweise Abtrennung von Aggregaten, denaturierten Proteinen oder/und Kontaminationen vor oder/und nach einer thermischen Behandlung, kann eine Fällung, z.B. mit Ammoniumsulfat, Ethanol oder Polyethylenglykol, verbunden mit einer Abtrennung der ausgefällten Aggregate nach bekannten Methoden, z.B. durch Zentrifugation, umfassen. Darüber hinaus kann die Abtrennung auch eine Gelfiltration umfassen z.B. mit Fractogel EMD Bio SEC (Merck) oder mit anderen Gelfiltrationsmedien wie etwa Sephadex oder Sepharose (Pharmacia). Vorzugsweise erfolgt die Abtrennung jedoch durch Membranfiltration unter Verwendung von Membranen geeigneter Porengröße, z.B. einer Ausschlußgröße von 30 bis 1000 kD und insbesondere von 100 bis 500 kD, wobei unerwünschte Bestandteile mit einem Molekulargewicht oberhalb der Ausschlußgröße abgetrennt werden. Weiterhin kann die Abtrennung auch eine Nanofiltration, wie sie zur Abreicherung von Viren eingesetzt wird, umfassen, z.B. unter Verwendung der Filtrationsmaterialien DV20 oder DV50 (Pall Corp., New York, USA) oder Planova 15 N (Asaki, Tokyo, JP).

Die vor oder vorzugsweise nach der Abtrennung durchgeführte thermische Behandlung umfaßt vorzugsweise eine Temperaturerhöhung über einen längeren Zeitraum, z.B. einen Pasteurisierungsschritt, der auf bekannte Weise erfolgen kann. Üblicherweise umfaßt die thermische Behandlung ein Erhitzen der Proteinlösung auf mindestens 55°C für eine ausreichende Dauer, um infektiöse Verunreinigungen zumindest weitgehend zu inaktivieren. Die Dauer des Erhitzens beträgt vorzugsweise mindestens 5 h, besonders bevorzugt ca. 10 h. Die Temperatur beim Erhitzen liegt vorzugsweise im Bereich zwischen 60 und 65°C. Um eine Inaktivierung der Proteine zu verhindern, wird die thermische Behandlung vorzugsweise in Gegenwart von bekannten Stabilisatoren wie etwa N-Acetyltryptophanat oder Natriumcaprylat im Falle von Albumin durchgeführt.

Während bei vielen Proteingemischen der oben genannte Abtrennungsschritt bereits für sich eine ausreichende Verringerung der Bildung von Aggregaten, denaturierten Proteinen oder/und Kontaminationen bewirkt, können in anderen Fällen die gewünschten Resultate nur dadurch erhalten werden, daß vor der Abtrennung ein Vorbehandlungsschritt durchgeführt wird, bei dem in der Proteinpräparation vorhandene aggregierbare oder/und denaturierbare Komponenten in einen abtrennbaren Zustand überführt werden. Vorzugsweise umfaßt der Vorbehandlungsschritt eine Veränderung physikalisch-chemischer Parameter in der Proteinpräparation, insbesondere eine Stressbehandlung, um in der Präparation bereits vorhandene denaturierte, teilweise denaturierte oder empfindliche Moleküle oder Kontaminationen zu aggregieren oder auf andere Weise in einen Zustand zu bringen, aus dem sie mit dem nachfolgenden Abtrennungsschritt entfernt werden können. Diese Veränderung physikalisch-chemischer Parameter kann beispielsweise eine Änderung des pH-Werts, eine Änderung, insbesondere eine Erhöhung, der Temperatur, eine Änderung der Ionenstärke oder die Dielektrizitätskonstante, das Zuführen von Scherkräften oder eine Kombination von zwei oder mehreren dieser Maßnahmen umfassen.

Besonders bevorzugt umfaßt dieser Vorbehandlungsschritt eine Erhöhung der Temperatur der Proteinpräparation. Das Ausmaß und die Dauer dieser Temperaturerhöhung sind von der jeweiligen Proteinpräparation bzw. deren Empfindlichkeit abhängig. Einerseits sollte die Temperaturerhöhrung und deren Dauer ausreichend sein, um einen möglichst großen Anteil aggregierbarer Komponenten in einen abtrennbaren Zustand überzuführen; andererseits dürfen die Bedingungen nicht zu drastisch gewählt werden, um eine Inaktivierung der erwünschten Proteine in der Präparation möglichst weitgehend zu vermeiden. Für eine Vielzahl von Proteinen, beispielsweise für Albumin hat es sich als günstig erwiesen, die Proteinpräparation für eine Dauer von 30 min bis 4 h, insbesondere für 1 h bis 2,5 h auf eine Temperatur von 40 bis 70°C, insbesondere von 45 bis 65°C zu erhitzen.

Das erfindungsgemäße Verfahren eignet sich besonders zur Herstellung von medizinischen Proteinpräparationen mit verringertem Aggregatgehalt. Vorzugsweise wird der Aggregatgehalt gegenüber einer - ansonsten gleichen - Proteinpräparation ohne den Abtrennungsschritt um mindestens 10%, besonders bevorzugt um mindestens 30% und am meisten bevorzugt um mindestens 50% verringert. In manchen Fällen wird sogar eine Verringerung des Aggregatgehalts um 90% oder darüber erzielt.

Schließlich betrifft die Erfindung auch noch eine Proteinpräparation mit verringertem Aggregatgehalt, die durch das erfindungsgemäße Verfahren hergestellt wurde. Diese Proteinpräparation ist insbesondere für medizinische Anwendungen erheblich besser geeignet als Präparate des Standes der Technik, da sie bei therapeutischer Anwendung erheblich weniger Unverträglichkeitareaktionen auslöst.

Schließlich wird die Erfindung noch durch die nachfolgenden Beispiele erläutert.

### Beispiele

### Beispiel 1

Eine Albuminlösung (10% in 10 mmol/l NaCl) wurde 90 min bei 45°C inkubiert. Anschließend wurde die Lösung durch eine Kassette mit einem Molekulargewicht-Cut Off von 300 kD diafiltriert. Das Ultrafiltrat, das vorwiegend monomeres Albumin enthält, wurde in Gegenwart von Stabilisatoren (16 mmol/l Na-Caprylat, 16 mmol/l Acetyltryptophan) 10 h lang bei 60°C pasteurisiert.

### Resultate:

Der Aggregatgehalt war nach der Vorinkubation 2%, nach der Ultrafiltation im (Ultrafiltrat) > 0,1% und nach dem Pasteurisieren 0,6%. Im Retentat der Ultrafiltration wurde eine starke Anreicherung der Aggregate gemessen (> 80%). Ohne Vorinkubation und anschließende Diafiltration wurde nach der Pasteruisation ein Aggregatgehalt von 6% gemessen. Die Resultate wurden nicht wesentlich durch Zugabe von Stabilisatoren (N-Acetyltryptophanat und Natriumcaprylat) während der Vorinkubation des Albumins beeinflußt.

Ein analoger Versuch wurde mit einer kommerziell erhältlichen Albuminlösung (20% in 140 mmol/l NaCl, 12 mmol/l N-Acetyltryptophanat und Na-Caprylat) durchgeführt. Das Verhalten der Lösungen während des Versuchs und die Resultate waren im Wesentlichen identisch wie mit der 10% Albuminlösung als Ausgangsmaterial.

### Bestimmung des Aggregatgehaltes in Lösungen:

1 mg Protein wurde mittels High Performance Gel Filtration auf einer Superose HR 10/30 Säule (Pharmacia) in 70 mmol/l Kaliumphosphat-Puffer, pH 7,0 mit einem Fluß von 1,0 ml/min aufgetrennt. Die Detektion erfolgte durch Bestimmung der Absorption bei 280 nm. Der Monomer- bzw. Aggregatgehalt der Proben wurde über die Flächen der Proteinpeaks berechnet.

### Beispiel 2

Eine Albuminlösung (10%, enthaltend je 8 mmol/l Stabilisatoren) wurde mit 1 mol/l NaOH auf pH 10,5 eingestellt und anschließend 2 h bei 65°C erwärmt. Danach wurde die Lösung auf Raumtemperatur abgekühlt und der pH-Wert mit 1 mol/l HCl auf 7 rücktitriert.

Mittels selektiver Fällung wurden die gebildeten Aggregate wie folgt entfernt:
(a) Die Lösung wurde mit Ammoniumsulfat bis zu einer Sättigung von 25%, 30% oder 35% versetzt. Die danach auftretende Trübung wurde mittels Zentrifugation entfernt und der Überstand mittels Gelfiltration auf Sephadex G-25 (PD-10, Pharmacia) in 150 mmol/l NaCl umäquilibriert. Anschließend wurde in Gegenwart von Stabilisator (Na-Caprylat, N-Acetyltryptophanat, je 8 mmol/l) bei 60°C während 10 h pasteurisiert.
(b) Die Lösung wurde mit Polyethylenglykol (PEG) 3000 bis zu einer Endkonzentration von 6,1%, 7,1% oder 8,3% versetzt und die damit verursachte Trübung mittels Zentrifugation entfernt. Der Überstand wurde anschließend wie unter (a) umäquilibriert und zur Gewinnung des Endprodukts in Gegenwart von Stabilisatoren pasteurisiert.

### Resultate:

| | | % **Aggregatgehalt im Endprodukt** | % **Abnahme der Aggregatbildung bez. Kontrolle** |
|---|---|---|---|
| **Albumin ohne Vorbehandlung (Kontrolle)** | | 22,9 | |
| **Ammoniumsulfat** | 25% | 15,8 | 31 |
| | 30% | 3,7 | 84 |
| | 35% | 2,8 | 88 |
| **PEG** | 6,1% | 20,5 | 10 |
| | 7,1% | 16,8 | 27 |
| | 8,3% | 9,3 | 59 |

### Beispiel 3

### Aktivierung von PEG:

5,5 g Cyanurchlorid wurden in 400 ml wasserfreiem Benzol enthaltend 10 g Natriumcarbonat gelöst. 19 g PEG 1900 wurden der Mischung zugegeben und über Nacht bei Raumtemperatur gerührt. Die Lösung wurde filtriert und langsam wurden 600 ml Petrolether zugegeben. Die Suspension wurde filtriert und das Präzipitat in 400 ml Benzol gelöst. Der Fällungs- und Filtrationsvorgang wurde mehrmals wiederholt, um freies Cyanurchlorid zu entfernen.

### Bindung des aktivierten PEG an Albumin:

1 g Albumin wurde in 100 ml 0,1 mol/l Natriumtetraborat, pH 9,2 gelöst. Bei 4°C wurden 8 g aktiviertes PEG zugegeben und der pH-Wert während einer Stunde bei 9,2 gehalten. 80 bis 90% der primären Aminogruppen wurden so mit PEG modifiziert. Nach der Reaktion wurde überschüssiges PEG mittels Diafiltration (10 kD Cut-Off Membran) entfernt und die Lösung gegen 10 mmol/l NaCl umgepuffert. Die Lösung wurde anschließend wie in Beispiel 1 bei 45°C vorinkubiert, durch eine 300 kD Kassette diafiltriert und in Gegenwart von Stabilisatoren bei 60°C 10 h pasteurisiert.

### Resultat:

Ohne Vorinkubation wurde im PEG-modifizierten Albumin ein Aggregatgehalt von 10% gemessen. Durch Vorinkubation und anschließende Diafiltration konnte der Aggregatgehalt auf 4,5% gesenkt werden.

### Beispiel 4

Eine 20%ige Albuminlösung wurde mit 0,1 mol/l Boratpuffer pH 9,5 auf 10% verdünnt und auf 0°C abgekühlt. Anschließend wurden 20% (v/v) kalte KJ₃ Lösung zugegeben und 30 min bei 0°C inkubiert. Die Reaktion wurde durch Zugabe von einigen Tropfen NaSO₃ (1 mol/l) gestoppt, die Proteinlösung 2 h bei 60°C inkubiert und abgekühlt. Ein Aliquot wurde für 8 h bei 60°C inkubiert (zur Beurteilung der Aggregatbildung).

Der Rest der Lösung wurde über eine 300 kD Membran gegen 4,5 Volumina 140 mmol/l NaCl diafiltriert. Zuletzt wurde das Retentat auf eine Proteinkonzentration von 20% eingeengt. Das Ultrafiltrat wurde mittels 10 kD Diafiltration umgepuffert: Diafiltration gegen 10 Volumina 140 mmol/l NaCl und anschließende Konzentrierung an der 10 kDa Diafiltrationsmembran auf einen Proteingehalt von 15 bis 20%. Die resultierende Proteinlösung wurde wie in Beispiel 1 in Gegenwart von Stabilisatoren pasteurisiert.

Mit Vorbehandlung (Vorinkubation, 300 kD Diafiltration) wurde im iodierten Albumin ein Aggregatgehalt von 5,2% erreicht, ohne die Vorbehandlung wurde ein Aggregatgehalt von 12,7% gemessen, d.h. es konnte eine Senkung um 59% erreicht werden.

### Beispiel 5

Albumin (20%) wurde mit gesättigter Na-Acetat Lösung pH 7,5 1:2 verdünnt und auf 0°C abgekühlt. Innerhalb 1 h wurde in Portionen Essigsäureanhydrid zudosiert (gleiches Gewicht wie das vorgelegte Protein). Anschließend wurde der pH-Wert auf 7,5 eingestellt, die Probe durch einen 1,2 µm Filter filtriert und mittels 10 kD Diafiltration umgepuffert (gegen 30 Volumina 140 mmol/l NaCl).

Die Proteinlösung wurde 2 h bei 60°C inkubiert, dann abgekühlt und über eine 300 kD Membran gegen 4 Volumina 140 mmol/l NaCl diafiltriert. Zuletzt wurde das Retentat auf ca. 20% eingeengt. Das Ultrafiltrat wurde mittels 10 kD Diafiltration umgepuffert (Diafiltration gegen 10 Volumina 150 mmol/l NaCl) und an der gleichen Membran auf einen Proteingehalt von 15-20% konzentriert. Die Lösung des acetylierten Albumins wurde anschließend wie in Beispiel 1 in Gegenwart von Stabilisatoren pasteurisiert.

Mit Vorbehandlung (Vorinkubation, 300 kD, Diafiltration) wurde im Endprodukt ein Aggregatgehalt von 62% gemessen, ohne die Vorbehandlung gelierte das acetylierte Produkt, d.h. es war kein lösliches Protein mehr vorhanden.

### Beispiel 6

Eine Albuminlösung (10%) mit 0,5 mmol/l EDTA wurde mit 0,1 M Perchlorsäure auf pH 3,2 eingestellt und auf 30°C erwärmt. Der Proteinlösung wurde H₂O₂ ad 0,5 mmol/l zugegeben. Anschließend wurde während 2 h bei 30°C inkubiert. Nach der Inkubation erfolgte eine 10 kD-Diafiltration gegen 4 Volumina 140 mmol/l NaCl. Das Retentat wurde über eine 300 kD Membran gegen 2 Volumina 140 mmol/l NaCl diafiltriert, zuletzt wurde das Retentat auf 15 bis 20% Proteinkonzentration eingeengt.

Das Ultrafiltrat wurde mittels 10 kD Diafiltration umgepuffert (10 Volumina 140 mmol/l NaCl) und an derselben Membran auf einen Gehalt von 10 bis 15% Protein konzentriert. Die Lösung des oxidierten Albumins wurde anschließend wie in Beispiel 1 in Gegenwart von Stabilisatoren pasteurisiert.

Mit Vorbehandlung (300 kD Diafiltration) wurde im Endprodukt ein Aggregatgehalt von 1,1% gemessen. Ohne die Vorbehandlung wies das oxidierte Albumin einen Aggregatgehalt von 11,1% auf. Der Aggregatgehalt konnte somit um 90% gesenkt werden.

### Beispiel 7

Eine Albuminlösung (10%, 20 ml) wurde mit NaOH auf pH 8,5 bis 9,0 eingestellt. Dazu wurden 50 mg Dimethyl-Suberimidat, suspendiert in 2 ml Triethanolamin bei pH 9,7 gegeben und 2 h bei Raumtemperatur inkubiert. Die Reaktion wurde durch Zugabe von 10% (v/v) 0,1 mol/l Tris pH 8,5 gestoppt. Die Proteinlösung wurde anschließend 2 h bei 60°C inkubiert und danach abgekühlt. 15 ml dieser Lösung wurden über eine 300 kD Membran diafiltriert (gegen 12 Volumina 140 mmol/l NaCl). Zuletzt wurde das Retentat möglichst stark eingeengt.

Das Ultrafiltrat wurde mittels 10 kD Diafiltration gegen 30 Volumina 140 mmol/l NaCl umgepuffert und anschließend mittels Vakuumdialyse gegen 140 mmol/l NaCl auf einen Gehalt von 15 bis 20% Protein konzentriert. Die Lösung des durch den Crosslinker modifizierten Albumins wurde anschließend wie in Beispiel 1 in Gegenwart von Stabilisatoren pasteurisiert.

Mit Vorbehandlung (Inkubation, 300 kD Diafiltration) wurde im Endprodukt ein Aggregatgehalt von 2,5% gemessen. Ohne die Vorbehandlung wies das modifizierte Albumin einen Aggregatgehalt von 20,5% auf. Der Aggregatgehalt konnte somit um 88% gesenkt werden.

### Beispiel 8

Einer Albuminlösung (10% Protein, in 10% Ethanol) wurden 4-(2-Bromacetamido)-2,2,6,6-tetramethylpiperidin-1-oxyl (BrAcTPO; gelöst in Ethanol, 500 mg/ml) zugegeben (0,177 g BrAcTPO/g Protein). Die Lösung wurde auf 45°C erwärmt, der pH-Wert wurde mit NaOH auf 9,5 eingestellt und durch kontinuierliche Zugabe von Lauge während 2 bis 3 h auf diesem Wert gehalten. Anschließend wurde der pH-Wert mit HCl auf 7,2 rücktitriert und die Lösung 90 min auf 60°C erwärmt. Danach erfolgte eine 300 kD Diafiltration mit 15 Volumina 140 mmol/l NaCl. Das Ultrafiltrat wurde mit 10 kD, 5 bis 10 Volumina 140 mmol/l NaCl diafiltriert und schließlich an derselben Membran auf einen Proteingehalt von 20% konzentriert. Die Lösung wurde in Gegenwart von Stabitisatoren wie unter Beispiel 1 pasteurisiert.

Mit Vorbehandlung (Inkubation, 300 kD Diafiltration) wurde im polynitroxylierten Albumin ein Aggregatgehalt von 0,1% gemessen. Ohne die Vorbehandlung wies das modifizierte Albumin einen Aggregatgehalt von 5,5% auf. Der Aggregatgehalt konnte somit um mehr als 90% gesenkt werden.

### Beispiel 9

Eine Lösung von Apolipoprotein A-l (10 g/l, in 10 mmol/NaCl) wurde bei pH 5,0 bei 60°C während 2 h inkubiert. Anschließend wurde der pH Wert auf 7,5 eingestellt und Guanidin-HCl zugegeben zu einer Konzentration von 2 mol/l und bei 45°C für 2 h inkubiert. Diese Lösung wurde über eine 300 kD Membran diafiltriert (10 Vol 10 mmol/l NaCl) und anschließend mit einer 10 kD Membran diafiltriert und auf 10 g/l aufkonzentriert.

Ohne Vorbehandlung (4 Versuche) wurden in den Apolipoprotein A-l Lösungen Aggregatgehalte von 2,4% bis 5,4% gemessen, mit Vorbehandlung (3 Versuche) konnte der Aggregatgehalt auf 0,4 bis 0,8% gesenkt werden.

### Beispiel 10

Eine Lösung von Immunglobulin G (20 g/l, ≤ 3 mmol NaCl/L) wurde bei pH 7,0, bei 45°C während 12 h inkubiert. Der Gehalt an Aggregaten stieg während der Vorbehandlung von < 0,1 auf 1,0%. Anschließend wurde über ein Acrylgel (Sepharyl S-300 HR) gelfiltriert, der pH-Wert mit HCl auf 5,3 eingestellt und mit einer 10 kD Membran auf 120 g/l aufkonzentriert. Der Aggregatgehalt lag bei 0,2% und blieb während der Lagerung während 6 Monaten bei 37°C stabil. In einer nicht behandelten Vergleichslösung stieg der Gehalt unter denselben Bedingungen auf 0,8%.

## Patentansprüche

1. Verfahren zur Herstellung von Proteinpräperationen mit verringertem Aggregatgehalt umfassend eine thermische Behandlung,
**dadurch gekennzeichnet,**
daß vor oder/und nach der thermischen Behandlung eine Abtrennung von in der Proteinpräparation vorhandenen Aggregaten, denaturierten Proteinen oder/und Kontaminationen erfolgt.

2. Verfahren nach Anspruch 1,
**dadurch gekennzeichnet,**
daß eine Präparation von Blutplasmaproteinen hergestellt wird.

3. Verfahren nach Anspruch 1 oder 2,
**dadurch gekennzeichnet,**
daß die Blutplasmaproteine ausgewählt werden aus der Gruppe bestehend aus Plasminogen, Albumin, Faktor VIII, Faktor IX, Fibronektin, Immunoglobulinen, Kininogen, Antithrombin III, α-1-Antitrypsin, Präkallikrein, Fibrinogen, Thrombin, (Apo-)Lipoproteinen und Blutplasmaproteinfraktionen, die ein oder mehrere dieser Proteine enthalten.

4. Verfahren nach einem der Ansprüche 1 bis 3,
**dadurch gekennzeichnet,**
daß eine Präparation von Albumin, Immunglobulin oder (Apo-)Lipoproteinen hergestellt wird.

5. Verfahren nach einem der Ansprüche 1 bis 4,
**dadurch gekennzeichnet,**
daßeine Präparation von chemisch modifizierten Proteinen hergestellt wird.

6. Verfahren nach Anspruch 5,
**dadurch gekennzeichnet,**
daß die chemische Modifizierung ausgewählt wird aus der Gruppe bestehend aus Polyethylenglykol-Modifizierung, Iodierung, Acylierung, Oxidation, Nitroxylierung und Quervernetzung.

7. Verfahren nach einem der Ansprüche 1 bis 6,
**dadurch gekennzeichnet,**
daß die thermische Behandlung ein Erhitzen der Proteinpräparation auf mindestens 55°C für eine ausreichende Dauer umfaßt, um infektiöse Verunreingungen zumindest weitgehend zu beseitigen.

8. Verfahren nach einem der Ansprüche 1 bis 7,
**dadurch gekennzeichnet,**
daß die Dauer der thermischen Behandlung mindestens 5 h beträgt.

9. Verfahren nach einem der Ansprüche 1 bis 8,
**dadurch gekennzeichnet,**
daß die thermische Behandlung in Gegenwart von Stabilisatoren durchgeführt wird.

10. Verfahren nach einem der Ansprüche 1 bis 9,
**dadurch gekennzeichnet,**
daß die Abtrennung einen Fällungsschritt umfaßt.

11. Verfahren nach Anspruch 10,
**dadurch gekennzeichnet,**
daß die Fällung durch Zugabe von Ammoniumsulfat, Ethanol oder Polyethylenglykol erfolgt.

12. Verfahren nach einem der Ansprüche 1 bis 11,
**dadurch gekennzeichnet,**
daß die Abtrennung einen Gelfiltrationsschritt umfaßt.

13. Verfahren nach einem der Ansprüche 1 bis 12,
**dadurch gekennzeichnet,**
daß die Abtrennung eine Nanofiltration umfaßt.

14. Verfahren nach einem der Ansprüche 1 bis 13,
**dadurch gekennzeichnet,**
daß die Abtrennung eine Membranfiltration umfaßt.

15. Verfahren nach Anspruch 14,
**dadurch gekennzeichnet,**
daß für die Membranfiltration eine Ausschlußgröße von 30 bis 1000 kD gewählt wird.

16. Verfahren nach einem der vorhergehenden Ansprüche,
**dadurch gekennzeichnet,**
daß vor Abtrennung ein Vorbehandlungsschritt durchgeführt wird, bei dem in der Proteinpräparation vorhandene aggregierbare oder/und denaturierbare Komponenten in einen abtrennbaren Zustand überführt werden.

17. Verfahren nach Anspruch 16,
**dadurch gekennzeichnet,**
daß der Vorbehandlungsschritt eine Veränderung physikalischchemischer Parameter in der Proteinpräparation umfaßt.

18. Verfahren nach Anspruch 17,
**dadurch gekennzeichnet,**
daß die Veränderung physikalisch-chemischer Parameter eine Änderung des pH-Werts, eine Erhöhung der Temperatur, eine Änderung der Ionenstärke oder der Dielektrizitätskonstante, das Zuführen von Scherkräften oder eine Kombination von zwei oder mehreren dieser Maßnahmen umfaßt.

19. Verfahren nach Anspruch 18,
**dadurch gekennzeichnet,**
daß die Temperatur der Proteinpräparation erhöht wird.

20. Verfahren nach Anspruch 19,
**dadurch gekennzeichnet,**
daß die Proteinpräparation für 30 min bis 4 h auf eine Temperatur von 40°C bis 70°C erhitzt wird.

21. Verfahren nach Anspruch 20,
**dadurch gekennzeichnet,**
daß die Proteinpräparation für 1 h bis 2,5 h auf eine Temperatur von 45-65°C erhitzt wird.

22. Verwendung des Verfahrens nach einem der Ansprüche 1 bis 21, zur Herstellung von medizinischen Proteinpräparationen mit verringertem Aggregatgehalt.

23. Verwendung nach Anspruch 22,
**dadurch gekennzeichnet,**
daß der Aggregatgehalt gegenüber einer Proteinpräparation ohne Abtrennungsschritt um mindestens 10% verringert wird.

24. Verwendung nach Anspruch 23,
**dadurch gekennzeichnet,**
daß der Aggregatgehalt um mindestens 50% verringert wird.

25. Proteinpräparation mitverringertem Aggregatgehalt hergestellt durch ein Verfahren nach einem der Ansprüche 1 bis 21.
